(19) Europäisches Patentamt
European Patent Office
Office européen des brevets

(11) **EP 2 750 778 B1**

(12) **FASCICULE DE BREVET EUROPEEN**

(45) Date de publication et mention de la délivrance du brevet:
**31.08.2022 Bulletin 2022/35**

(21) Numéro de dépôt: **12761665.4**

(22) Date de dépôt: **30.07.2012**

(51) Classification Internationale des Brevets (IPC):
**B01D 21/28** *(2006.01)*   **B01D 21/34** *(2006.01)*
**B01D 43/00** *(2006.01)*   **F15D 1/00** *(2006.01)*
**G01N 35/00** *(2006.01)*

(52) Classification Coopérative des Brevets (CPC):
**B01D 21/283; B01D 21/34;** B01D 2221/10

(86) Numéro de dépôt international:
**PCT/IB2012/053882**

(87) Numéro de publication internationale:
**WO 2013/030691 (07.03.2013 Gazette 2013/10)**

(54) **DISPOSITIFS ET PROCÉDÉS DE MANIPULATION D'OBJETS PAR CHAMP DE FORCE ACOUSTIQUE**

VORRICHTUNGEN UND VERFAHREN ZUR HANDHABUNG VON GEGENSTÄNDEN MITTELS AKUSTISCHER KRAFTFELDER

DEVICES AND METHODS FOR HANDLING OBJECTS, USING ACOUSTIC FORCE FIELDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.08.2011 FR 1157658**

(43) Date de publication de la demande:
**09.07.2014 Bulletin 2014/28**

(73) Titulaires:
• **Centre National de la Recherche Scientifique 75794 Paris Cedex 16 (FR)**
• **École Supérieure de Physique et de Chimie Industrielles de la Ville de Paris 75231 Paris Cedex 05 (FR)**

(72) Inventeurs:
• **AIDER, Jean-Luc 92170 Vanves (FR)**
• **DRON, Olivier 91440 Bures-sur-Yvette (FR)**
• **HOYOS, Mauricio 94000 Créteil (FR)**

(74) Mandataire: **Icosa 83 avenue Denfert-Rochereau 75014 Paris (FR)**

(56) Documents cités:
WO-A1-02/072234   WO-A1-2006/032703
WO-A1-2011/152796   WO-A2-2006/032048

## Description

La présente invention concerne les dispositifs de manipulation d'objets par mise en œuvre d'un champ de force acoustique au sein d'un canal, notamment d'un micro-canal. En particulier, la présente invention concerne un dispositif destiné à la manipulation d'objets présents dans un canal au sein d'un fluide, notamment d'un liquide, ledit dispositif comprenant un générateur d'ondes acoustiques configuré pour fournir une fréquence f différente d'une fréquence f0 de résonance du canal le long de son deuxième axe transversal, ledit générateur d'ondes acoustiques est un générateur d'ondes acoustiques à bande large, et ledit générateur d'ondes acoustiques n'utilisant pas la fréquence f0 de résonance du canal le long du deuxième axe transversal.

## Arrière-plan

L'acoustophorèse permet de manipuler et trier des particules par le biais d'une force acoustique. La technique classique connue de l'état de l'art consiste à créer au moins un nœud de pression acoustique à une position déterminée le long d'une dimension (longueur, largeur ou épaisseur) d'un canal en assurant une condition de résonance pour l'onde acoustique.

La présente invention permet de s'affranchir de la contrainte sur la position du nœud de pression imposée par la condition de résonance.

WO 2006/095117 décrit un dispositif de séparation fluidique dans lequel un champ de force acoustique peut être généré.

WO 98/17373 et WO 02/072234 décrivent des méthodes de séparation de particules par application d'un champ de force acoustique aboutissant à la formation d'une onde stationnaire au sein d'un canal.

La publication Dron et al. « Parametric study of acoustic focusing of particles in a micro-channel in the perspective to improve micro-PIV measurements » (Microfluid Nano-fluid (2009) 7 : 857-867) décrit l'intérêt de la focalisation acoustique par formation d'une onde stationnaire dans un canal afin d'améliorer des mesures par micro-PIV.

WO 2009/071733 décrit la focalisation de particules sur la largeur d'un micro-canal par mise en œuvre d'un transducteur acoustique opérant à des fréquences correspondant à des fréquences de résonance dudit micro-canal.

WO 2006/032703 décrit une méthode de séparation de particules par modification (« switch ») de la fréquence appliquée d'une fréquence de résonance d'un canal à une autre.

La publication Glynne-Jones et al. « Mode-switching: A new technique for electronically varying the agglomération position in an acoustic particle manipulator » (Ultrasonics 50 (2010) 68-75) décrit la possibilité de déplacer la hauteur de focalisation acoustique sur une partie de la hauteur d'un canal par modification rapide de la fréquence de travail d'une fréquence de résonance dudit canal à une autre.

La publication Svennebring et al. « Selective Bioparticle Rétention and Characterization in a Chip-Integrated Confocal Ultrasonic Cavity » (J. Biotech. Bioeng, 103, 323-328 (2009)) décrit la possibilité de manipuler des cellules en opérant à une fréquence de résonance d'une cavité.

La publication Petersson et al. « Séparation of lipids from blood utilizing ultrasonic standing waves in microfluidic channels » (Analyst, 2004, 129, 938-943) décrit la séparation des lipides du sang grâce à la formation d'une onde acoustique stationnaire.

La publication Lipkens et al. « The Effect of Frequency Sweeping and Fluid Flow on Particle Trajectories in Ultrasonic Standing Waves » (IEEE Sensors Journal, Vol. 8, No.6, Juin 2008, 667-677) décrit une simulation de l'effet produit par une variation de la fréquence de travail d'un transducteur générant un champ de force acoustique suivant la longueur d'un macro-canal.

De nombreuses méthodes d'acoustophorèse connues à ce jour sont fortement limitées par la position des nœuds de pression qui est imposée par la condition de résonance.

En cas de facteur de contraste d'impédance acoustique négatif (bulle, lipide, liposome), les particules vont migrer vers les ventres de pression.

On connaît, par ailleurs, WO 2004/030800 qui décrit l'utilisation d'un dispositif permettant de promouvoir des phénomènes de mélange dans un canal mais qui ne décrit aucunement un phénomène assimilable à de la focalisation acoustique.

En outre, les méthodes connues peuvent ne pas être en mesure de focaliser de manière satisfaisante, au sein d'un canal, une grande quantité d'objets et notamment des nappes d'objets.

Il existe, par conséquent, un besoin pour un dispositif permettant une focalisation d'objets sur l'intégralité d'une dimension d'un canal et pas seulement en des positions discrètes correspondant aux nœuds ou aux ventres de pression obtenus lorsqu'il est opéré à une fréquence de résonance du canal.

Il existe un besoin pour obtenir un dispositif permettant de focaliser, au sein d'un canal, de grandes quantités d'objets et notamment des nappes d'objets.

Il existe un besoin pour obtenir un dispositif permettant de faire varier la position de focalisation des objets en fonction d'une caractéristique et/ou de la position de ces derniers au sein d'un canal.

Il existe encore un besoin pour obtenir un dispositif permettant une mesure améliorée de la norme et/ou de la direction et/ou du sens du vecteur vitesse d'objets présents au sein d'un canal.

La présente invention vise à répondre à tout ou partie des besoins précités.

## Résumé

Selon un premier de ses aspects, l'invention concerne un dispositif destiné à la manipulation d'objets présents dans un canal au sein d'un fluide, notamment d'un liquide, comportant :

- un canal s'étendant suivant un axe longitudinal, le canal ayant une section transversale présentant une largeur mesurée suivant un premier axe transversal et une épaisseur mesurée selon un deuxième axe transversal perpendiculaire au premier, la largeur étant supérieure ou égale à l'épaisseur, le canal comportant, suivant le deuxième axe transversal, des première et deuxième parois,
- un générateur d'ondes acoustiques générant des ondes acoustiques dans le canal depuis au moins l'une desdites parois,

  ledit générateur d'ondes acoustiques étant configuré pour fournir une fréquence f différente d'une fréquence $f_0$ de résonance du canal le long du deuxième axe transversal,
  ledit générateur d'ondes acoustiques étant un générateur d'ondes acoustiques à bande large, et
  ledit générateur d'ondes acoustiques n'utilisant pas la fréquence f0 de résonance du canal (2) le long du deuxième axe transversal (Z).

L'invention est exposée dans le jeu de revendications joint.

Par « axe longitudinal du canal », il faut comprendre la ligne joignant l'ensemble des barycentres des sections transversales du canal. L'axe longitudinal du canal peut être rectiligne ou courbe et peut être contenu dans un plan, lequel peut être un plan de symétrie pour certaines, voire toutes les sections transversales du canal.

L'épaisseur $\underline{e}$ du canal correspond à la distance, mesurée selon le deuxième axe transversal, séparant les première et deuxième parois.

Par « $f_0$ étant une fréquence de résonance du canal le long du deuxième axe transversal », il faut comprendre que $f_0$ est telle que l'épaisseur $\underline{e}$ du canal, mesurée au niveau d'une position donnée le long de l'axe longitudinal du canal, vérifie

$$e = \frac{n\lambda}{2}$$

où n est un nombre entier et

$$\lambda = \frac{c_f}{f_0}$$

où $c_f$ désigne la vitesse du son dans le fluide présent au sein du canal, à la température du fluide, par exemple 20°C. En d'autres termes, la fréquence $f_0$ correspond à la fréquence théorique satisfaisant, au niveau d'une position donnée le long de l'axe longitudinal du canal, la condition de résonance de l'onde acoustique dans le canal et l'obtention d'une onde stationnaire le long de son deuxième axe transversal, c'est-à-dire sur son épaisseur.

Les inventeurs ont mis en évidence expérimentalement la possibilité de faire varier la position de focalisation acoustique sur une partie importante de l'épaisseur du canal. Cet effet a été obtenu en générant une onde acoustique dans l'épaisseur dudit canal et en faisant varier la fréquence de travail du générateur d'ondes acoustiques entre différentes fréquences, chacune distincte d'une fréquence de résonance dudit canal le long de son deuxième axe transversal.

Sans vouloir être liés à aucune explication ou théorie, les inventeurs considèrent que le fait de générer un champ de force acoustique sur l'épaisseur et non sur la largeur, comme dans WO 2009/071733, peut permettre de limiter l'effet de courant acoustique (« acoustic streaming »).

La présente invention permet, par conséquent, de s'affranchir de la limitation à des emplacements discrets pour les positions de focalisation et constitue, de ce fait, une innovation majeure dans le domaine de la manipulation d'objets au sein d'un canal par mise en œuvre d'un champ de force acoustique.

Avantageusement, au moins une nappe d'objets est formée par focalisation acoustique.

Avantageusement encore, au moins un extremum de pression acoustique est formé au sein du fluide par les ondes acoustiques générées.

La nappe d'objets est de préférence focalisée au niveau d'un extremum de pression acoustique (nœud ou ventre acoustique) formé au sein du fluide par les ondes acoustiques générées. Une pluralité de nappes d'objets distinctes est par exemple formée, chacune de ces nappes étant présente au niveau d'un extremum de pression acoustique distinct.

La nappe d'objets formée peut avoir une forme allongée le long de l'axe longitudinal du canal étant par exemple de forme ovale ou rectangulaire lorsqu'observée selon une direction perpendiculaire au plan d'aplatissement de la nappe. En variante, la nappe d'objets formée peut avoir une forme circulaire ou carrée lorsqu'observée selon une direction perpendiculaire à son plan d'aplatissement.

### Générateur d'ondes acoustiques

Le générateur d'ondes acoustiques peut, par exemple, opérer à une fréquence inférieure ou égale à 10 MHz et notamment comprise entre 0,5 et 10 MHz.

L'utilisation du générateur d'ondes acoustiques dans de telles plages de fréquence peut avantageusement permettre de manipuler des cellules vivantes sans les endommager.

Le générateur d'ondes acoustiques opère, de préférence, à une fréquence f différente de $f_0$ et comprise entre $0,75f_0$ et $1,25f_0$, notamment entre 0,75fo et $0,95f_0$ ou entre $1,05f_0$ et $1,25f_0$.

L'utilisation du générateur d'ondes acoustiques dans ces plages de fréquence, proches d'une fréquence de résonance, peut avantageusement permettre de créer une force acoustique suffisamment élevée pour permettre une focalisation satisfaisante des particules.

Le générateur d'ondes acoustiques est un générateur d'ondes acoustiques à bande large.

Une pluralité de générateurs d'ondes acoustiques peuvent être disposés le long du canal et générer des ondes acoustiques depuis au moins l'une des première et deuxième parois, lesdits générateurs d'ondes acoustiques pouvant notamment être disposés du même côté du canal.

La mise en œuvre d'une pluralité de générateurs d'ondes acoustiques est avantageuse lorsque le fluide s'écoule à grande vitesse ou lorsque l'on veut générer des nappes de particules de grandes dimensions. Dans le premier cas, plus la vitesse du fluide est importante, plus le temps de vol sous les générateurs est court. Cela peut nécessiter la mise en œuvre d'un plus grand nombre de transducteurs afin d'obtenir la focalisation. Dans le second cas, par exemple en l'absence d'écoulement, on pourra utiliser plusieurs générateurs d'ondes acoustiques pour former des nappes de particules de grande taille.

Lorsque plusieurs générateurs d'ondes acoustiques sont utilisés, au moins l'un d'entre eux peut générer une onde acoustique le long du premier axe transversal du canal, c'est-à-dire le long de la largeur du canal.

Dans ce dernier cas, le rapport largeur/épaisseur peut être compris entre 1 et 10, notamment entre 1 et 3.

Le fait d'exercer un champ force acoustique sur l'épaisseur et sur la largeur peut avantageusement permettre de déplacer un ensemble de particules, par exemple une ligne de particules, dans une zone quelconque du canal et ainsi de bénéficier d'un plus grand nombre de lieux disponibles pour la focalisation acoustique.

Le générateur d'ondes acoustiques peut être alimenté par une tension sinusoïdale. En variante, le générateur d'ondes acoustiques peut être alimenté par une tension triangulaire ou en créneaux.

Le générateur d'ondes acoustiques peut être à commande numérique ou analogique.

Le générateur d'ondes acoustiques peut, par exemple, être fixé à la (aux) première et deuxième paroi(s) du canal. Cette fixation peut s'effectuer par tout moyen connu de l'homme du métier et notamment par collage.

Une couche de matériau d'adaptation acoustique peut être présente entre le générateur d'ondes acoustiques et au moins l'une des première et deuxième parois du canal.

L'adaptation acoustique peut être assurée par l'emploi de tout matériau connu par l'homme du métier comme adéquat pour ce faire.

Canal

L'épaisseur du canal peut, lorsque l'on se déplace le long de l'axe longitudinal de ce dernier être constante ou variable, avec par exemple au moins deux zones se succédant axialement et présentant des épaisseurs différentes.

Le canal peut, par exemple, avoir, sur au moins une portion de sa longueur, notamment sur la totalité de sa longueur, une épaisseur inférieure à 3 cm, mieux inférieure à 1 cm. Le canal est, par exemple, un micro-canal.

Par « micro-canal », il faut comprendre un canal ayant, sur la totalité de sa longueur, une épaisseur inférieure ou égale à 1 mm.

Le canal peut présenter sur au moins une portion de sa longueur, notamment sur la totalité de sa longueur, une épaisseur comprise entre 50 $\mu$m et 1 mm, de préférence entre 100 $\mu$m et 500 $\mu$m.

La largeur du canal peut, lorsque l'on se déplace le long de l'axe longitudinal de ce dernier, être constante ou variable, avec par exemple au moins deux zones se succédant axialement et présentant des largeurs différentes.

Le canal peut présenter sur au moins une portion de sa longueur, notamment sur la totalité de sa longueur, une largeur comprise entre 1 mm et 30 mm, de préférence entre 5 mm et 20 mm.

Le canal peut comporter une section transversale sensiblement constante lorsque l'on se déplace le long de son axe longitudinal.

Le canal peut présenter sur au moins une portion de sa longueur, notamment sur la totalité de sa longueur, une section transversale rectangulaire.

La longueur du canal, mesurée suivant l'axe longitudinal, peut, par exemple, être comprise entre 3 mm et 10 cm, de préférence entre 10 mm et 70 mm.

Le canal peut avantageusement présenter un rapport longueur/épaisseur supérieur ou égal à 10, par exemple supérieur ou égal à 12.

Au moins l'une des première et deuxième parois, de préférence les deux, peut comporter, notamment consister en, un matériau choisi parmi : les verres minéraux ou organiques, le quartz, les matériaux thermoplastiques, notamment le PMMA ou le polycarbonate, les métaux. On peut, plus généralement, utiliser tout matériau ayant une impédance acoustique élevée, c'est-à-dire au moins dix fois supérieure à celle du fluide.

La paroi en regard de celle depuis laquelle les ondes acoustiques sont générées peut comporter, notamment consister en, un matériau d'impédance acoustique au moins dix fois supérieure à celle du fluide.

L'utilisation au sein des parois de matériaux ayant une impédance acoustique élevée permet avantageusement d'améliorer la focalisation acoustique des objets en favorisant l'obtention d'un extremum de pression marqué.

Dans un exemple de réalisation, au moins l'une des première et deuxième parois, de préférence les deux, peut comporter, notamment consister en, un verre minéral ou organique ou du PMMA.

Le dispositif peut, par exemple, être tel que le générateur d'ondes acoustiques génère des ondes acoustiques depuis la première paroi du canal, laquelle peut être une paroi supérieure et :

- la première et la deuxième parois comportent, notamment consistent en, du PMMA, ou
- la première paroi et la deuxième paroi comportent, notamment consistent en, un verre minéral ou organique, ou
- la première paroi comporte, notamment consiste en, du PMMA et la deuxième paroi comporte, notamment consiste en, un verre minéral ou organique.

Les première et/ou deuxième parois peuvent, par exemple, être sous forme de plaques.

Les première et/ou deuxième parois peuvent présenter sur au moins une partie, notamment sur la totalité, de leur longueur une épaisseur comprise entre 0,5 mm et 5 mm.

Au moins l'une des première et deuxième parois, par exemple les deux, peu(ven)t être opaques.

En variante, au moins l'une des première et deuxième parois, par exemple les deux, peu(ven)t être transparentes. On peut privilégier la mise en œuvre de parois transparentes lorsque l'on cherche à acquérir des images d'objets présents au sein du canal comme détaillé ci-dessous.

La(es) paroi(s) opposée(s) à(aux) paroi(s) depuis la(es)quelle(s) les ondes acoustiques sont générées peu(ven)t osciller librement lorsque le dispositif est en fonctionnement.

Le canal peut comporter une pluralité de sorties vers lesquelles sont sélectivement dirigés les objets selon la fréquence f à laquelle opère le générateur d'ondes acoustiques.

La taille de la (des) sortie(s) vers la(es)quelle(s) sont sélectivement dirigés les objets peut être adaptée à la taille desdits objets.

## Fluides et objets

Le fluide peut être un liquide biologique comme par exemple du sang.

En variante, le fluide peut être de l'eau.

Le fluide peut, par exemple, être transparent au rayonnement visible.

Le fluide peut être au repos lorsque le dispositif est en fonctionnement. En variante, le fluide peut, lorsque le dispositif est en fonctionnement, être en écoulement, par exemple en écoulement laminaire.

Les objets peuvent, par exemple, être des cellules biologiques mono ou polydisperses, notamment des cellules sanguines, par exemple des globules. Dans ce dernier cas, le procédé selon l'invention peut, par exemple, être utilisé dans des méthodes de tri desdites cellules biologiques.

Les objets peuvent être des particules rigides ou déformables, par exemple des particules de polystyrène.

La taille moyenne des objets présents au sein du canal peut, par exemple, être inférieure ou égale à 50 μm. Par « taille moyenne », on désigne la dimension granulométrique statistique à la moitié de la population, dite D50.

La fréquence f à laquelle opère le générateur d'ondes acoustiques peut être telle que sa longueur d'onde associée soit supérieure à la taille moyenne des objets présents au sein du canal, de préférence supérieure ou égale à dix fois cette taille moyenne.

L'épaisseur du canal peut, au moins au niveau d'une position le long de l'axe longitudinal au niveau de laquelle sont générées les ondes acoustiques, être supérieure ou égale à dix fois la taille moyenne des objets présents au sein du canal.

## Capteur et système de contrôle

L'invention peut, dans un exemple de réalisation, concerner un ensemble comportant un dispositif tel que défini ci-dessus auquel sont associés :

- un capteur permettant de mesurer au moins une caractéristique et/ou la position des objets présents au sein du canal, ledit capteur générant, en fonction du résultat de cette mesure, un signal, et
- un système de contrôle recevant ledit signal et contrôlant la fréquence f à laquelle opère le générateur d'ondes acoustiques et/ou l'amplitude des ondes acoustiques générées en fonction dudit signal.

Le capteur peut encore permettre de mesurer au moins une caractéristique du fluide, notamment sa vitesse d'écoulement et/ou son débit et/ou sa température, ledit capteur générant, en fonction du résultat de cette mesure, un signal et le système de contrôle recevant ledit signal et contrôlant la fréquence f à laquelle opère le générateur d'ondes acoustiques et/ou l'amplitude des ondes acoustiques générées en fonction dudit signal.

Lorsque le fluide présent dans le canal est en écoulement, le capteur peut, par exemple, être placé en amont d'au moins un générateur d'ondes acoustiques relativement au sens de l'écoulement.

Le capteur peut, en variante, être placé en aval d'au moins un générateur d'ondes acoustiques relativement au sens de l'écoulement.

Le capteur peut, par exemple, permettre de mesurer la taille des objets. Dans ce cas, le capteur peut comporter :

- une source lumineuse, notamment un laser, destinée à illuminer les objets situés dans une zone déterminée du canal, et

- un système de contrôle comportant, notamment consistant en, un détecteur de rayonnement lumineux, destiné à détecter le rayonnement lumineux, issu de ladite source lumineuse et diffusé par lesdits objets, et apte à produire un signal lequel est fonction de la taille des objets ayant diffusé le rayonnement lumineux.

Selon un autre exemple de réalisation, la concentration des objets au sein d'une zone déterminée du canal peut être mesurée par le capteur.

Le capteur peut, par exemple, être un compteur Coulter ou un détecteur UV.

Le système de contrôle peut comporter un ordinateur.

Le système de contrôle peut contrôler un étage d'alimentation du générateur d'ondes acoustiques, par exemple un générateur de signal, relié à un étage amplificateur.

Acquisition et traitement d'images des objets présents au sein du canal

L'invention peut, dans un exemple de réalisation, concerner un ensemble comportant :

- un dispositif tel que défini plus haut,
- un système d'illumination configuré pour illuminer au moins une partie des objets présents au sein du canal, et
- un système d'acquisition d'images configuré pour acquérir au moins une image d'au moins une partie des objets présents au sein du canal et illuminés par le système d'illumination,

ledit ensemble comportant notamment un dispositif de traitement de la au moins une image produite par le système d'acquisition.

Le système d'illumination peut être configuré pour illuminer tout ou partie d'une nappe d'objets obtenue par focalisation acoustique.

Le dispositif de traitement peut permettre de mesurer la norme et/ou la direction et/ou le sens du vecteur vitesse d'au moins une partie des objets présents au sein du canal et illuminés par le système d'illumination.

L'ensemble selon l'invention peut notamment permettre de mettre en œuvre un procédé de vélocimétrie par image de particules (« PIV »).

Le dispositif de traitement de la au moins une image peut, par exemple, comporter un ordinateur.

Le dispositif de traitement d'image peut, par exemple, être configuré pour calculer un coefficient de corrélation des distributions d'intensité lumineuse obtenues sur au moins deux images d'objets produites par le système d'acquisition d'images.

Les inventeurs ont constaté que l'utilisation des dispositifs et méthodes de focalisation acoustique décrits dans la présente invention permettaient d'améliorer les mesures réalisées par vélocimétrie par image de particules en permettant notamment une focalisation acoustique des objets au sein du canal à une position précise sur toute l'épaisseur du canal.

Indépendamment ou en combinaison avec ce qui précède, la présente invention concerne, selon un autre de ses aspects, un dispositif destiné à la manipulation d'objets présents dans un canal au sein d'un fluide, notamment d'un liquide, comportant :

- un canal s'étendant suivant un axe longitudinal, le canal ayant une section transversale présentant une largeur mesurée suivant un premier axe transversal et une épaisseur mesurée selon un deuxième axe transversal perpendiculaire au premier, la largeur étant supérieure ou égale à l'épaisseur, le canal comportant, suivant le deuxième axe transversal, des première et deuxième parois, et
- un générateur d'ondes acoustiques à bande large générant des ondes acoustiques depuis au moins l'une des première et deuxième parois.

Procédés

Indépendamment ou en combinaison avec ce qui précède, la présente invention concerne, selon un autre de ses aspects, un procédé de manipulation d'objets présents dans un canal à l'aide d'un générateur d'ondes acoustiques, mettant notamment en œuvre un dispositif ou un ensemble tel que défini plus haut, dans lequel :

- ledit canal s'étend suivant un axe longitudinal et a une section transversale présentant une largeur mesurée suivant un premier axe transversal et une épaisseur mesurée selon un deuxième axe transversal perpendiculaire au premier, la largeur étant supérieure ou égale à l'épaisseur, le canal comportant, suivant le deuxième axe transversal, des première et deuxième parois, et
- ledit générateur d'ondes acoustiques génère des ondes acoustiques depuis au moins l'une des première et deuxième parois et opère à une fréquence f différente d'une fréquence $f_0$ de résonance du canal le long du deuxième axe transversal.

Le procédé décrit ci-dessus peut être utilisé dans l'une au moins des applications suivantes : méthodes de tri d'espèces, par exemple de particules rigides ou déformables, de particules polydisperses, de cellules biologiques, notamment de cellules sanguines, par exemple de cellules cancéreuses présentes dans un échantillon de sang ou de globules, de bactéries, d'émulsions colloïdales ou non colloïdales, de protéines ou de liposomes, méthodes de diagnostic ou d'analyse, méthodes de purification, d'enrichissement ou d'appauvrissement d'espèces, méthodes de synthèse d'espèces, méthodes de modification de caractéristiques physiques ou chimiques d'espèces, méthodes de recherche de médicaments, méthodes de mélange ou méthodes de mesure de coefficient de diffusion.

Le procédé selon l'invention peut, en particulier, être utilisé aux fins de séparation de particules comprises initialement dans un mélange de particules polydisperses.

Les différences de taille entre les particules polydisperses peuvent permettre la séparation de ces dernières du fait de leurs différences de vitesse de migration vers le nœud de pression acoustique généré le long de l'épaisseur du canal.

Le procédé selon l'invention peut permettre la formation d'au moins une nappe d'objets, par focalisation acoustique. Il est, en particulier, possible que le procédé selon l'invention comporte une étape consistant à faire réagir au moins deux espèces chimiques présentes au sein de la nappe formée par focalisation acoustique.

Le procédé selon l'invention peut encore permettre la coalescence de plusieurs nappes d'objets ou la fusion de films.

Le procédé selon l'invention peut encore permettre d'opérer une filtration sans filtre du fait de la focalisation acoustique sélective des objets manipulés.

Indépendamment ou en combinaison avec ce qui précède, l'invention concerne, selon encore un autre de ses aspects, un procédé de manipulation d'objets présents dans un canal à l'aide d'un générateur d'ondes acoustiques, mettant notamment en œuvre un dispositif tel que décrit plus haut, dans lequel :

- ledit canal s'étend suivant un axe longitudinal et a une section transversale présentant une largeur mesurée suivant un premier axe transversal et une épaisseur mesurée selon un deuxième axe transversal perpendiculaire au premier, la largeur étant supérieure ou égale à l'épaisseur, le canal comportant, suivant le deuxième axe transversal, des première et deuxième parois,
- ledit générateur d'ondes acoustiques génère des ondes acoustiques depuis au moins l'une des première et deuxième parois et opère à une fréquence f,
- au moins une caractéristique et/ou la position des objets présents au sein du canal est mesurée par un capteur,
- ledit capteur génère en fonction du résultat de ladite mesure un signal,
- ledit signal est acheminé vers un système de contrôle permettant de contrôler la fréquence à laquelle opère le générateur d'ondes acoustiques et/ou l'amplitude des ondes acoustiques générées en fonction dudit signal, et
- la fréquence f à laquelle opère le générateur d'ondes acoustiques est modifiée en fonction dudit signal.

Le procédé selon l'invention peut comporter une étape de déplacement des objets le long de l'épaisseur du canal suite à la modification, en fonction du signal généré par le système de contrôle, de la fréquence f à laquelle opère le générateur d'ondes acoustiques.

Le procédé selon l'invention peut ainsi avantageusement permettre de modifier en temps réel la position des objets par exemple selon la taille ou la nature de ces derniers.

Ainsi, un procédé selon l'invention de séparation de particules polydisperses pourra, par exemple, être réalisé en modifiant la fréquence à laquelle opère le générateur d'ondes acoustiques et en utilisant le fait que des particules de tailles différentes ont une vitesse de relaxation vers les nœuds différente.

Ledit déplacement des objets peut avoir lieu entre une première position, distincte d'une position de focalisation acoustique desdits objets, et une deuxième position, distincte de la première, laquelle est une position de focalisation acoustique desdits objets. Un tel déplacement peut notamment avoir lieu lorsque la mesure effectuée par le capteur est réalisée alors que les objets ne sont pas encore soumis aux ondes acoustiques générées.

En variante, le déplacement des objets, du fait de la modification de la fréquence f à laquelle opère le générateur d'ondes acoustiques, peut avoir lieu entre une première position de focalisation acoustique desdits objets et une deuxième position de focalisation acoustique desdits objets, ladite deuxième position étant distincte de la première.

La modification de la position des objets peut, lorsque le fluide est en écoulement, permettre de diriger sélectivement lesdits objets vers une sortie déterminée du canal.

En d'autres termes, le procédé selon l'invention peut permettre au moins en fonction du résultat d'une mesure d'une caractéristique et/ou de la position des objets au sein du canal de modifier la position de ces derniers.

L'étape de déplacement des objets du fait de la modification de la fréquence f à laquelle opère le générateur d'ondes acoustiques peut, par exemple, permettre de concentrer à une position déterminée des objets lesquels ont notamment sensiblement la même taille.

Une telle étape de concentration peut notamment être utilisée dans le cadre d'un procédé de tri d'espèces et notamment de tri de particules polydisperses.

Une telle étape de concentration peut encore être suivie d'au moins une réaction chimique, ladite réaction chimique pouvant permettre notamment de quantifier la teneur et/ou de déterminer la nature des objets.

La réaction chimique peut, par exemple, avoir lieu entre au moins deux composés ayant été concentrés au sein de la même position durant l'étape de concentration.

La réaction chimique peut avoir lieu au sein du canal, notamment au niveau du champ de force acoustique.

En variante, la réaction chimique peut ne pas avoir lieu au sein du canal. Dans ce cas, les objets concentrés par le procédé selon l'invention peuvent être sélectivement dirigés vers une sortie du canal afin, par exemple, d'être recueillis dans une enceinte. La réaction chimique peut alors avoir lieu dans ladite enceinte. En variante, l'enceinte comportant les objets concentrés par le procédé selon l'invention peut être transportée vers un dispositif annexe comportant un réactif destiné à quantifier la teneur et/ou à déterminer la nature desdits objets.

Comme mentionné plus haut, le capteur peut être placé en aval d'au moins un générateur d'ondes acoustiques relativement au sens de l'écoulement.

Dans ce cas, le procédé selon l'invention peut comporter les étapes consistant à :

- faire varier la fréquence à laquelle opère le générateur d'ondes acoustiques entre une pluralité de fréquences,
- mesurer par le capteur, pour chacune desdites fréquences, au moins une caractéristique et/ou la position des objets,
- comparer les valeurs obtenues pour les mesures effectuées à chacune desdites fréquences avec au moins une valeur de référence,
- sélectionner une fréquence parmi ladite pluralité de fréquence pour laquelle la comparaison des valeurs obtenues et de la valeur de référence fournit un résultat prédéterminé,
- faire opérer le générateur d'ondes acoustiques à cette fréquence sélectionnée.

Il est, par exemple, possible de mesurer en aval du générateur d'ondes acoustiques relativement au sens de l'écoulement la densité des objets et de sélectionner la fréquence pour laquelle on obtient une densité maximale.

Le procédé selon l'invention peut ainsi avantageusement comporter une étape d'apprentissage d'une fréquence optimale de fonctionnement et de contrôle du générateur d'ondes acoustiques pour que ce dernier opère à cette fréquence optimale.

On peut répéter cette phase d'apprentissage plusieurs fois dans le procédé selon l'invention ou, en variante, ne la mettre en œuvre qu'une fois ou pas du tout.

Indépendamment ou en combinaison avec ce qui précède, l'invention concerne un procédé d'acquisition d'au moins une image d'objets présents au sein d'un canal comportant les étapes consistant à :

a) manipuler les objets par mise en œuvre d'un procédé tel que décrit plus haut afin d'obtenir une focalisation acoustique desdits objets au niveau d'une zone déterminée du canal,
b) illuminer les objets au niveau de la zone de focalisation acoustique par l'intermédiaire d'un système d'illumination, et
c) acquérir, par l'intermédiaire d'un système d'acquisition, au moins une image desdits objets ainsi illuminés.

Le procédé peut par exemple comporter une étape d'acquisition d'au moins une première image des objets, à un premier instant, et d'une seconde image des objets, à un second instant.

Le procédé peut, en outre, comporter une étape de calcul d'un coefficient de corrélation des distributions d'intensité lumineuse obtenues sur ces première et seconde images.

Le procédé peut, en particulier, être un procédé de mesure de la norme et/ou de la direction et/ou du sens du vecteur vitesse d'objets présents au sein du canal et illuminés par le système d'illumination.

Ce procédé peut, par exemple, mettre en œuvre une étape de calcul d'une des grandeurs précitées à partir des première et seconde images définies ci-dessus.

Le procédé peut, par exemple, être un procédé de vélocimétrie par image de particules (« Particle Image Velocimetry »).

**Description des figures**

L'invention pourra être mieux comprise à la lecture de la description détaillée qui va suivre, d'exemples non limitatifs de mise en œuvre de celle-ci, et à l'examen du dessin annexé, sur lequel :

- la figure 1 représente, de manière schématique et partielle, un exemple de dispositif expérimental permettant de caractériser un générateur d'ondes acoustiques à bande large,
- la figure 2 représente, de manière schématique et partielle, un exemple de signal obtenu pour un générateur d'ondes acoustiques à bande large,
- la figure 3 représente, de manière schématique et partielle, un exemple de dispositif selon l'invention,
- la figure 4 représente, de manière schématique et partielle, une section selon IV-IV du dispositif de la figure 3,
- les figures 5 à 7 représentent, de manière schématique et partielle, des variantes de réalisation de l'invention,
- la figure 8 représente l'influence de la fréquence de l'onde acoustique sur la hauteur de focalisation des objets dans un micro-canal, et
- les figures 9 à 13 représentent l'influence de divers paramètres sur la focalisation acoustique.

**Protocole de caractérisation d'un générateur d'ondes acoustiques à bande large**

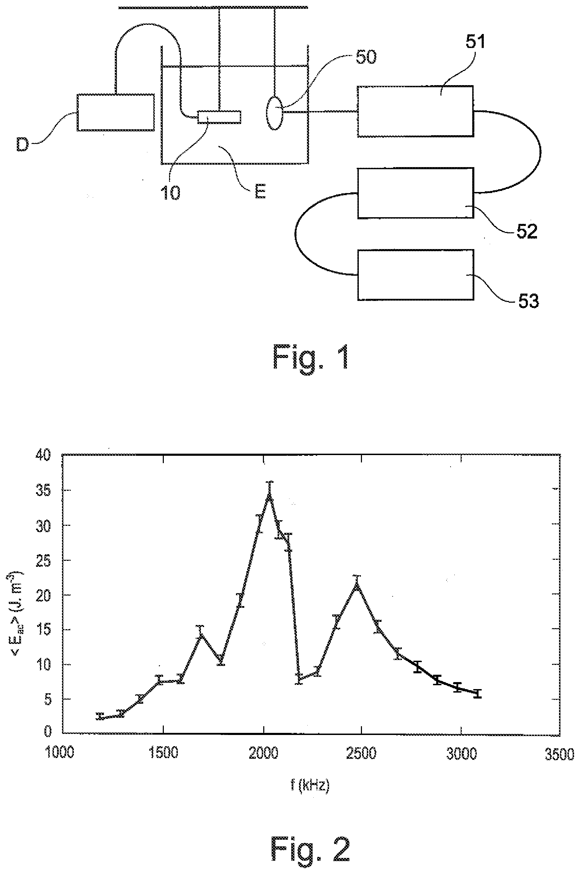

Le dispositif expérimental détaillé ci-dessous et représenté à la figure 1 permet de déterminer si un générateur d'ondes acoustiques peut être considéré comme étant à bande large.

Comme représenté à la figure 1, un générateur d'ondes acoustiques 10, opérant à une fréquence de travail donnée, est placé dans un bassin rempli d'eau E et alimenté par une tension sinusoïdale par un dispositif d'alimentation D. La tension d'alimentation a une amplitude de 10 V. Une membrane 50 de polyéthylène terephtalate (Mylar®) est placée en regard du générateur d'ondes acoustiques 10, la membrane 50 étant perpendiculaire à l'axe du générateur d'ondes acoustiques 10. La membrane 50 est choisie, notamment de par son épaisseur et son espacement avec le générateur d'ondes acoustiques, afin que le déplacement de cette dernière corresponde au déplacement des particules de fluide du fait de la génération de l'onde acoustique. En d'autres termes, la membrane 50 oppose une résistance négligeable à l'écoulement des particules de fluide crée par l'onde acoustique produite.

Le faisceau d'un laser (non représenté) est dirigé vers la membrane 50 et réfléchi par cette dernière. Le faisceau réfléchi par la membrane 50 est ensuite dirigé vers un photodétecteur 51 lequel transmet un signal proportionnel à l'intensité lumineuse reçue. Le signal obtenu en sortie du photodétecteur 51 est ensuite démodulé par un démodulateur 52 afin d'obtenir une tension qui est une fonction linéaire du déplacement de la membrane 50.

Cette tension est ensuite différenciée numériquement par rapport au temps en utilisant une fréquence d'échantillonnage de 5 GHz par l'intermédiaire d'un dispositif 53 prévu à cet effet. La différenciation numérique peut, par exemple, être effectuée par un logiciel de simulation numérique comme Matlab®.

On obtient alors le profil de vitesse de déplacement de la membrane en fonction du temps et la valeur maximale de ce profil de vitesse de déplacement de la membrane vo est relevé et moyenné sur 10 mesures afin d'obtenir v'o. On en déduit, ensuite, la valeur moyenne de l'énergie acoustique <Eac> laquelle est donnée par la formule :

$$< E_{ac} > = \frac{\rho_F \, v o'^2}{2}$$

où $\rho_F$ désigne la densité du fluide.

Afin de déterminer si le générateur d'ondes acoustiques est à bande large, on quantifie l'influence de la variation de fréquence à laquelle opère le générateur d'ondes acoustiques sur la grandeur <Eac>. On réitère le protocole décrit ci-dessus en faisant varier la fréquence de travail du générateur d'ondes acoustiques. On reporte alors, pour les différentes valeurs de fréquence, la valeur <Eac> obtenue. La valeur maximale d'énergie acoustique $<E_{ac}>_{max}$ sera obtenue pour la fréquence nominale de travail du générateur d'ondes acoustiques.

On considère que le générateur d'ondes acoustiques est à bande large lorsque l'on obtient un rapport $<E_{ac}> / <E_{ac}>_{max}$ supérieur ou égal à 15%, de préférence supérieur ou égal à 40 %, sur une plage de fréquence [0,75*fréquence nominale du générateur d'ondes acoustiques ; 1,25*fréquence nominale du générateur d'ondes acoustiques. On a représenté, à la figure 2, l'évolution de <Eac> en fonction de la fréquence pour un générateur d'ondes acoustiques à bande large obtenue dans les conditions opératoires détaillées ci-dessus.

Dans cet exemple, la membrane de polyéthylène terephtalate a une épaisseur de 12 $\mu$m et est située à une distance de 1 mm du générateur d'ondes acoustiques.

Dans l'exemple de la figure 2, le générateur d'ondes acoustiques utilisé est un transducteur commercialisé par la société Signal processing®, de géométrie cylindrique, de longueur 30 mm, de diamètre 7 mm et de fréquence nominale de travail d'environ 2 MHz.

## Exemples de dispositifs utilisés dans le cadre de la présente invention

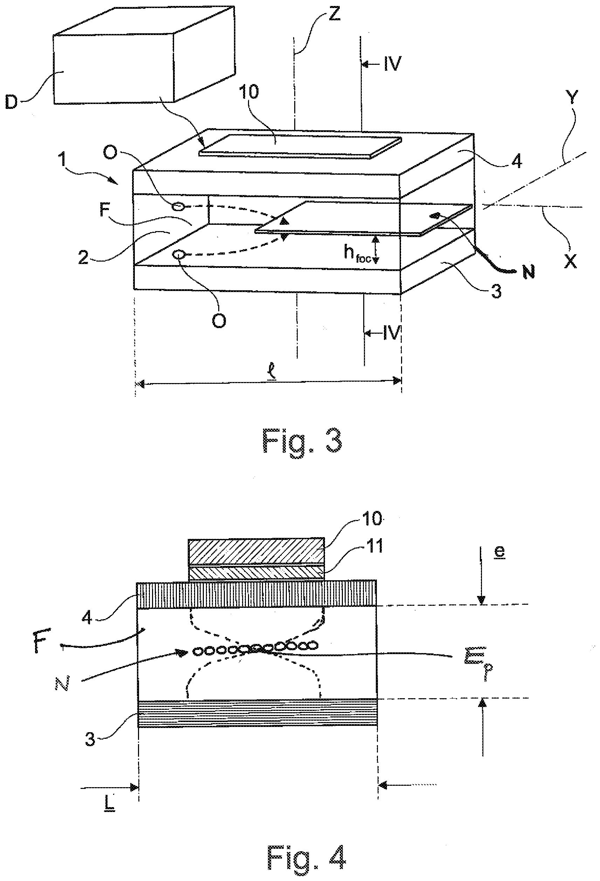

La figure 3 représente un dispositif 1 selon l'invention. Ce dispositif comporte une paroi inférieure 3 et une paroi supérieure 4 délimitant un canal 2 dans lequel un fluide F est contenu. Le fluide F peut être en écoulement, par exemple en écoulement laminaire ou, en variante, être au repos.

Le fluide F comporte une pluralité d'objets O, lesquels peuvent être mono ou polydisperses. Les objets O peuvent, en particulier, être des cellules biologiques et le fluide F peut alors être un liquide biologique comme, par exemple, du sang.

Un transducteur acoustique 10 peut, comme représenté, être fixé à la paroi supérieure 4 du dispositif 1. Le transducteur 10 est un transducteur à bande large.

Le transducteur 10 peut, en variante, ne pas être un transducteur à bande large tant qu'il permet de générer des ondes acoustiques à une fréquence f différente d'une fréquence de résonance $f_o$ du canal 2 le long du deuxième axe transversal Z.

Le transducteur commercialisé par la société Signal processing®, de géométrie cylindrique, de longueur 30 mm, de diamètre 7 mm et de fréquence nominale de travail d'environ 2 MHz peut, par exemple, être mis en œuvre dans le dispositif 1 selon l'invention.

Il est possible d'utiliser un transducteur 10 ayant une autre géométrie que cylindrique, notamment une géométrie parallélépipédique comme représenté à la figure 3.

Le transducteur 10 est alimenté par un signal provenant d'un générateur D lequel peut, par exemple, comporter, notamment consister en, un générateur de signal connecté en série avec un étage amplificateur de tension. Le générateur D, de préférence, alimente le transducteur 10 avec un signal sinusoïdal de fréquence déterminée. En variante, la tension d'alimentation peut être triangulaire ou en créneaux.

Lors de son fonctionnement, le transducteur 10 permet d'obtenir un champ de force acoustique le long de l'axe transversal Z du canal suivant l'épaisseur de ce dernier et ainsi de permettre à des objets O de se focaliser à une altitude de focalisation $h_{foc}$. Comme mentionné plus haut, l'altitude de focalisation $h_{foc}$ est fonction de la fréquence de travail du transducteur 10.

La figure 3 illustre la possibilité d'obtenir une nappe N d'objets O à la hauteur de focalisation $h_{foc}$. Cette nappe N peut être formée au niveau d'un extremum $E_p$ de pression (nœud ou ventre) généré par le transducteur 10 (voir figure 4).

La paroi 3 comporte, dans l'exemple illustré à la figure 3, un matériau d'impédance acoustique élevée, c'est-à-dire au moins dix fois supérieure à celle du fluide F. On peut, par conséquent, avoir une paroi 3 en regard de la paroi 4 depuis laquelle les ondes acoustiques sont générées qui comporte un matériau d'impédance acoustique élevé. Deux parois sont dites « en regard » lorsqu'elles sont situées le long de l'axe d'application des ondes acoustiques générées par le transducteur 10 fonctionnant à une fréquence f différente de $f_0$.

Dans une variante de réalisation, chacune des parois 3 et 4 comporte, notamment consiste en, un matériau d'impédance acoustique élevée.

Comme représenté à la figure 4, une couche de gel 11 permettant une adaptation d'impédances acoustiques peut être présente entre le transducteur 10 et la paroi supérieure 4.

On a, par ailleurs, représenté à la figure 4 la formation d'un extremum $E_p$ de pression au sein du fluide F par les ondes acoustiques générées par le transducteur 10. Dans l'exemple illustré, la nappe N d'objets O est focalisée au niveau de l'extremum $E_p$ de pression.

La nappe N d'objets O peut être focalisée au niveau d'un nœud de pression, comme illustré. Dans une variante non illustrée, la nappe N est focalisée au niveau d'un ventre de pression. Dans une variante non illustrée, les ondes acoustiques générées forment une pluralité d'extrema de pression acoustique, et deux nappes distinctes sont chacune focalisées au niveau d'un extremum de pression distinct. On peut, par conséquent, obtenir une première nappe focalisée au niveau d'un premier extremum de pression et une deuxième nappe focalisée au niveau d'un deuxième extremum de pression, différent du premier.

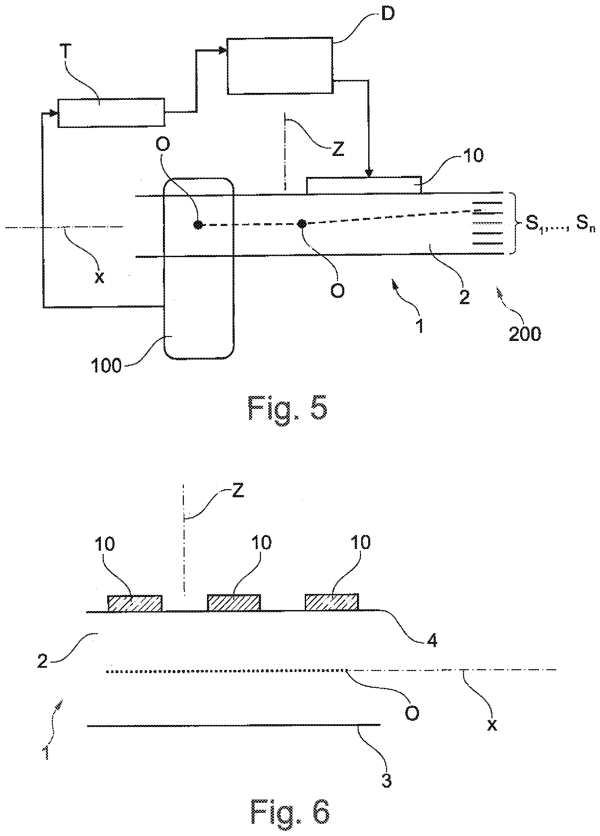

On a représenté à la figure 5, un exemple de réalisation d'un ensemble 200 comportant un dispositif 1 selon l'invention et un capteur 100 permettant de mesurer au moins une caractéristique et/ou la position des objets présents au sein du canal 2. Le capteur 100 génère, en fonction du résultat de cette mesure, un signal acheminé vers un système de contrôle T. Le système de contrôle T permet, en fonction du signal provenant du capteur 100, d'agir sur le générateur D afin de faire varier la fréquence à laquelle opère le transducteur 10 et/ou l'amplitude des ondes acoustiques générées.

Le capteur 100 peut, par exemple, permettre de mesurer la densité et/ou la taille des objets O.

Le signal généré par le capteur 100 peut avoir pour conséquence de contrôler le transducteur 10 afin de diriger sélectivement les objets O au niveau d'au moins une des sorties ($S_1$,..., $S_n$).

Le capteur 100 peut, par exemple, permettre de mesurer la taille des objets O et comporter, pour ce faire, un laser et un détecteur mesurant l'intensité lumineuse diffusée par les objets O présents au sein du canal 2.

Le capteur 100 peut, en variante, comporter, notamment consister en, un compteur Coulter, permettant de compter les objets O et de déterminer leur taille, ou un détecteur UV

On peut, dans un exemple de réalisation, former des nappes de particules ou de cellules ou même des membranes biologiques et les diriger vers l'une des sorties ($S_1$,..., Sn).

On a représenté à la figure 6 un exemple de réalisation de dispositif 1 comportant une pluralité de transducteurs 10. Dans cet exemple, les transducteurs 10 sont disposés le long du canal 2 du même côté.

Il est encore possible, dans le cadre de la présente invention, que les transducteurs 10 soient disposés de part et d'autre du canal 2.

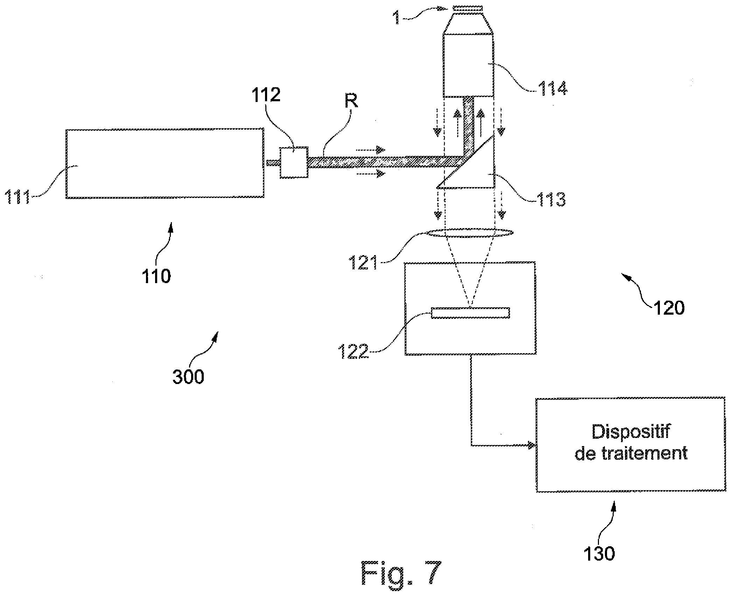

On a représenté à la figure 7 un exemple de réalisation d'un ensemble 300 selon l'invention permettant l'acquisition d'une image d'objets O présents au sein du canal 2. L'ensemble 300 comporte un dispositif 1 associé à un système d'illumination 110 ainsi qu'à un système d'acquisition d'images 120.

Le système d'illumination 110 comporte une source lumineuse 111 laquelle peut par exemple comporter un laser et notamment un laser Nd : YAG. Dans un mode de réalisation, la source lumineuse 111 comporte une association de deux lasers Nd : YAG pulsés et les objets O sont des particules fluorescentes, les lasers Nd : YAG émettant un rayonnement de longueur d'onde 532 nm destiné à être absorbé par les objets O.

Une structure optique 112 peut être placée à la sortie de la source lumineuse 111 afin d'adapter le rayonnement issu de celle-ci au dispositif optique.

Le rayonnement R produit à la sortie de la structure 112 peut être dirigé vers une structure réfléchissante 113 afin d'être focalisé vers une lentille 114.

Le séparateur 113 peut, par exemple, comporter une association d'un filtre ainsi qu'un miroir dichroïque. La lentille 114 peut, par exemple, être une lentille de microscope dont le plan focal peut être situé sensiblement au niveau de la zone de focalisation acoustique.

La structure réfléchissante 113 peut être choisie notamment de manière à ne pas filtrer le rayonnement de longueur d'onde apte à être absorbé par les objets O présents au sein du canal 2 par exemple lorsque les objets O sont fluorescents.

Par exemple, lorsque les objets O sont fluorescents, le rayonnement R peut être absorbé et les objets O peuvent émettre un rayonnement de longueur d'onde différente par exemple supérieure.

La structure réfléchissante 113 peut être configurée pour ne laisser substantiellement passer que le rayonnement réémis par les objets O dirigé vers la lentille 121.

Le dispositif d'acquisition d'images 120 comporte quant à lui une lentille 121 laquelle permet de focaliser le rayonnement provenant des objets O présents au sein du canal 2 sur un capteur 122 permettant de produire une image desdits objets O. Le capteur 122 peut par exemple être une caméra CCD.

Le capteur 122 peut par exemple être connecté à un dispositif de traitement d'images 130, lequel peut comporter un ordinateur.

Le dispositif de traitement 130 peut permettre de mesurer la norme et/ou la direction et/ou le sens du vecteur vitesse d'au moins une partie des objets O présents au sein du canal 2 et illuminés par le système d'illumination 110.

Lorsqu'au moins deux images des objets O sont prises, le dispositif de traitement 130 peut permettre de calculer un coefficient de corrélation des distributions d'intensité lumineuse obtenues sur ces au moins deux images d'objets O.

**Exemples**

Exemple 1

Le canal utilisé est un micro-canal d'épaisseur 337 $\mu$m, de largeur 10 mm et de longueur 40 mm. Le micro-canal est rempli d'eau. Les parois supérieure et inférieure du micro-canal sont toutes les deux sous forme de plaques de 1 mm d'épaisseur et constituées de PMMA. Les objets sont des particules de polystyrène de diamètre 7 $\mu$m et leur concentration est de 56 mg/L.

Ce type de canal présente deux pics de résonance autour de 2 MHz et 2,5 MHz.

Le générateur d'ondes acoustiques utilisé est un transducteur cylindrique commercialisé par la société Signal processing® ayant un diamètre de 7 mm, une hauteur de 30 mm ainsi qu'une fréquence nominale de travail d'approximativement 2 MHz. Le générateur d'ondes acoustiques est fixé au niveau de la paroi supérieure du micro-canal. La tension d'alimentation du transducteur est de 10 V.

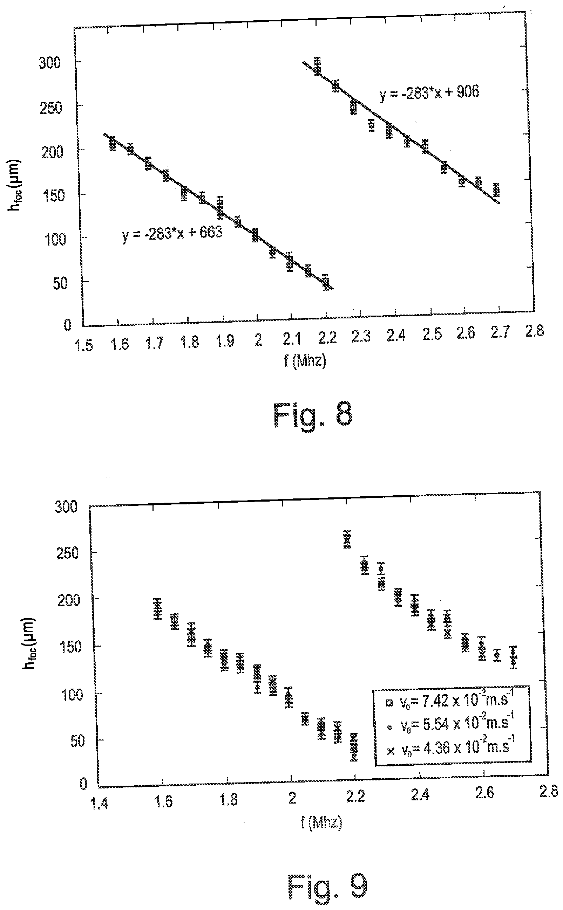

On a représenté à la figure 8 l'influence de la fréquence de travail du transducteur sur la hauteur de focalisation des particules $h_{foc}$. Sur cette figure ont été superposées les courbes obtenues avec ou sans étape de ré-homogénéisation des particules au sein du micro-canal entre l'application de deux fréquences distinctes.

Exemple 2 : influence de l'énergie acoustique

Les conditions opératoires détaillées dans l'exemple 1 ont été reprises à l'exception de la tension d'alimentation du transducteur dont l'influence a été examinée. Trois expérimentations ont été réalisées correspondant chacune à des tensions d'alimentation du transducteur de 5 V, 7 V et 10 V. Les valeurs maximales du profil de vitesse des particules de fluide du fait de la génération de l'onde acoustique sont reportées sur la figure 9. Les résultats obtenus sont représentés en figure 9. Il apparaît que l'amplitude de l'onde acoustique, commandée par la tension d'alimentation du transducteur, n'a pas d'impact sur le phénomène, mis en évidence, de déplacement de la hauteur de focalisation des particules.

Exemple 3: influence de la taille des objets

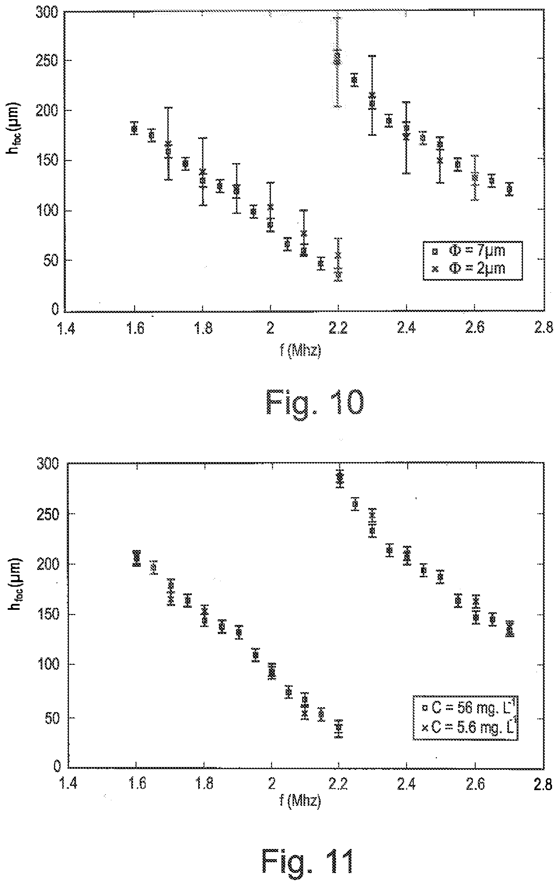

Les conditions opératoires détaillées dans l'exemple 1 ont été reprises à l'exception du diamètre des particules dont l'influence a été examinée. Deux expérimentations ont été réalisées correspondant chacune à l'utilisation de particules de diamètre 2 $\mu$m et 7 $\mu$m. Les résultats obtenus sont représentés en figure 10. Il apparaît que le diamètre des particules utilisées n'a pas d'impact sur le phénomène, mis en évidence, de déplacement de la hauteur de focalisation des particules.

Exemple 4: influence de la concentration des objets

Les conditions opératoires détaillées dans l'exemple 1 ont été reprises à l'exception de la concentration en objets dont l'influence a été examinée. Deux expérimentations ont été réalisées correspondant chacune à la mise en œuvre de particules à des concentrations de 5,6 mg/L et 56 mg/L. Les résultats obtenus sont représentés en figure 11. Il apparaît que la concentration en particules mise en œuvre n'a pas d'impact sur le phénomène, mis en évidence, de déplacement de la hauteur de focalisation des particules.

Exemple 5 : influence du matériau constituant les parois

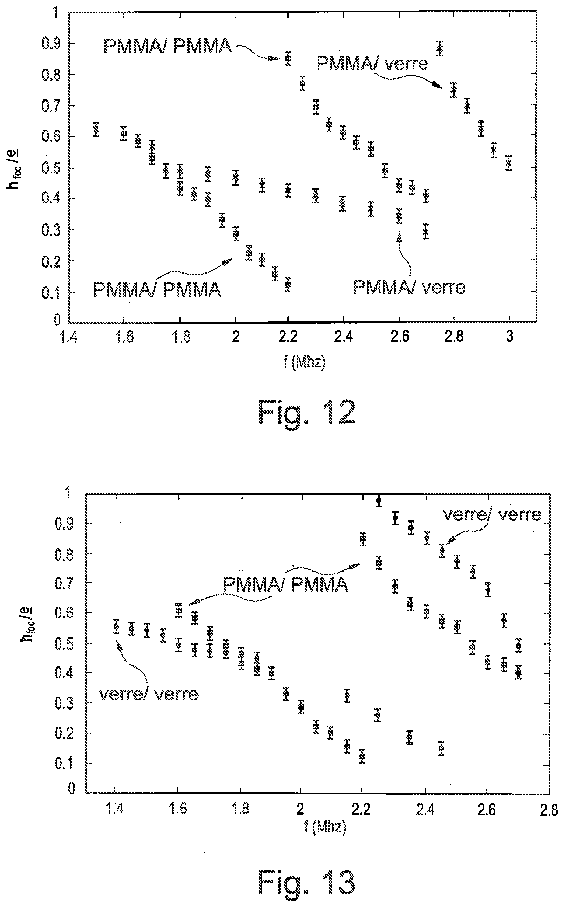

Les conditions opératoires détaillées dans l'exemple 1 ont été reprises à l'exception de la nature des matériaux constituant les parois supérieures et inférieures dont l'influence a été examinée. L'épaisseur du micro-canal a, par ailleurs, à chaque fois été adaptée à la nature des matériaux utilisés pour constituer les parois. Les résultats obtenus sont représentés en figures 12 et 13.

L'annotation « PMMA/PMMA » correspond à un canal ayant des parois supérieure et inférieure constituées de PMMA. L'annotation « PMMA/verre » correspond à un canal ayant une paroi supérieure en PMMA et une paroi inférieure en verre. L'annotation « verre/verre » correspond à un canal ayant des parois supérieure et inférieure constituées de verre.

L'expression « comportant un(e) » doit être comprise comme « comportant au moins un(e) ».

Sauf indications contraires, l'expression « compris(e) entre » doit s'entendre comme bornes incluses.

**Revendications**

1. Dispositif (1) destiné à la manipulation d'objets (O) présents dans un canal (2) au sein d'un fluide (F), notamment d'un liquide, comportant :

- un canal (2) s'étendant suivant un axe longitudinal (X), le canal (2) ayant une section transversale présentant une largeur (L) mesurée suivant un premier axe transversal (Y) et une épaisseur (e) mesurée selon un deuxième axe transversal (Z) perpendiculaire au premier, la largeur (L) étant supérieure ou égale à l'épaisseur (e), le canal comportant, suivant le deuxième axe transversal (Z), des première (3) et deuxième (4) parois,
- un générateur d'ondes acoustiques (10) générant des ondes acoustiques dans le canal depuis au moins l'une desdites parois (3 ; 4),

**caractérisé en ce que** :

ledit générateur d'ondes acoustiques (10) est configuré pour fournir une fréquence f différente d'une fréquence $f_0$ de résonance du canal (2) le long du deuxième axe transversal (Z),
ledit générateur d'ondes acoustiques (10) est un générateur d'ondes acoustiques à bande large, et
ledit générateur d'ondes acoustiques (10) n'utilise pas la fréquence $f_0$ de résonance du canal (2) le long du deuxième axe transversal (Z).

2. Dispositif (1) selon la revendication **1,** le canal (2) ayant, sur au moins une portion de sa longueur, notamment sur la totalité de sa longueur, une épaisseur (e) inférieure à 3 cm, notamment inférieure à 1 cm, le canal (2) étant de préférence un micro-canal.

3. Dispositif (1) selon l'une quelconque des revendications **1** et **2,** le canal (2) présentant, sur au moins une portion de sa longueur, notamment sur la totalité de sa longueur, une section transversale rectangulaire.

4. Dispositif (1) selon l'une quelconque des revendications précédentes, comportant une pluralité de générateurs d'ondes acoustiques (10) disposés le long du canal et générant dans le canal des ondes acoustiques depuis au moins l'une des parois (3 ; 4), lesdits générateurs d'ondes acoustiques (10) étant notamment disposés d'un même côté du canal (2).

5. Dispositif (1) selon l'une quelconque des revendications précédentes, au moins l'une des parois (3 ; 4), de préférence les deux, comportant, notamment consistant en, un matériau choisi parmi : les verres minéraux ou organiques, les matériaux thermoplastiques notamment le polyméthacrylate de méthyle ou le polycarbonate, le quartz, les métaux dont le produit (densité du métal x vitesse du son dans ce métal) est supérieur ou égal à $10^6$ Pa.s/m.

6. Dispositif (1) selon l'une quelconque des revendications précédentes, au moins une nappe (N) d'objets (O) étant formée par focalisation acoustique, notamment au moins un extremum ($E_p$) de pression acoustique étant formé au sein du fluide (F) par les ondes acoustiques générées, et la nappe (N) d'objets (O) étant focalisée au niveau de l'extremum (Ep) de pression acoustique.

7. Dispositif selon l'une quelconque des revendications précédentes, le générateur d'ondes acoustiques (10) opérant à une fréquence f différente de $f_0$ et comprise entre $0,75f_0$ et $1,25f_0$, notamment entre $0,75f_0$ et $0,95f_0$ ou entre $1,05f_0$ et $1,25f_0$.

8. Dispositif (1) selon la revendication **7,** les première et deuxième parois comportant un matériau ayant une impédance acoustique au moins dix fois supérieure à celle du fluide.

9. Dispositif (1) selon l'une quelconque des revendications précédentes, le canal (2) comportant une pluralité de sorties ($S_1$,..., $S_n$) vers lesquelles sont sélectivement dirigés les objets (O) selon la fréquence f à laquelle opère le générateur d'ondes acoustiques (10).

10. Ensemble (200) comportant :

- un dispositif (1) selon l'une quelconque des revendications **1** à **9,**
- un capteur (100) permettant de mesurer au moins une caractéristique et/ou la position des objets (O) présents au sein du canal (2), ledit capteur (100) générant, en fonction du résultat de cette mesure, un signal, et
- un système de contrôle (T) recevant ledit signal et contrôlant la fréquence f à laquelle opère le générateur d'ondes acoustiques (10) et/ou l'amplitude des ondes acoustiques générées en fonction dudit signal.

11. Ensemble (300) comportant :

- un dispositif (1) selon l'une quelconque des revendications **1** à **9,**
- un système d'illumination (110) configuré pour illuminer au moins une partie des objets (O) présents au sein du canal (2), et
- un système d'acquisition d'images (120) configuré pour acquérir au moins une image d'au moins une partie des objets (O) présents au sein du canal (2) et illuminés par le système d'illumination (110),

ledit ensemble comportant notamment un dispositif de traitement (130) de la au moins une image acquise par le système d'acquisition (120),

le dispositif de traitement (130) permettant de mesurer la norme et/ou la direction et/ou le sens du vecteur vitesse d'au moins une partie des objets (O) présents au sein du canal (2) et illuminés par le système d'illumination (110).

12. Procédé de manipulation d'objets (O) présents dans un canal (2) à l'aide d'un générateur d'ondes acoustiques (10), mettant en œuvre un dispositif (1) selon l'une quelconque des revendications **1** à **9** ou un ensemble selon l'une quelconque des revendications **10** à **11,** procédé dans lequel :

- ledit canal (2) s'étend suivant un axe longitudinal (X) et a une section transversale présentant une largeur (L) mesurée suivant un premier axe transversal (Y) et une épaisseur (e) mesurée selon un deuxième axe transversal (Z) perpendiculaire au premier, la largeur (L) étant supérieure ou égale à l'épaisseur (e), le canal comportant, suivant le deuxième axe transversal (Z), des première (3) et deuxième (4) parois, et
- ledit générateur d'ondes acoustiques (10) génère des ondes acoustiques dans le canal depuis au moins l'une des parois (3 ; 4) et opère à une fréquence f différente d'une fréquence $f_0$ de résonance du canal (2) le long du deuxième axe transversal (Z),
- au moins une caractéristique et/ou la position des objets (O) présents au sein du canal (2) est mesurée par un capteur (100),
- un signal est généré en fonction du résultat de la mesure réalisée par ledit capteur (100) et est acheminé vers un système de contrôle (T), et
- la fréquence f à laquelle opère le générateur d'ondes acoustiques (10) et/ou l'amplitude des ondes acoustiques générées est modifiée en fonction dudit signal par action du système de contrôle (T).

13. Procédé d'acquisition d'au moins une image d'objets (O) présents au sein d'un canal (2) comportant les étapes consistant à :

a) manipuler les objets (O) par mise en œuvre d'un procédé selon la revendication **12** afin d'obtenir une focalisation acoustique desdits objets (O) au niveau d'une zone déterminée du canal (2),
b) illuminer les objets (O) au niveau de la zone de focalisation acoustique par l'intermédiaire d'un système d'illumination (110), et
c) acquérir, par l'intermédiaire d'un système d'acquisition (120), au moins une image desdits objets (O) ainsi illuminés.

14. Procédé de mesure de la norme et/ou de la direction et/ou du sens du vecteur vitesse d'objets (O) présents au sein d'un canal (2) comportant les étapes consistant à :

- acquérir une première image des objets (O), à un premier instant, par mise en œuvre du procédé selon la revendication précédente,
- acquérir une seconde image des objets (O), à un second instant, par mise en œuvre du procédé selon la revendication précédente, et
- calculer à partir des première et seconde images une mesure de la norme et/ou de la direction et/ou du sens du vecteur vitesse des objets (O).

15. Procédé selon l'une quelconque des revendications **12** à **14, caractérisé par le fait qu'**il est utilisé dans l'une au moins des applications suivantes : vélocimétrie par image de particules (« PIV »), notamment vélocimétrie par image de particules micrométriques (« micro-PIV »), méthodes de tri d'espèces, par exemple de particules rigides ou déformables, de particules polydisperses, de cellules biologiques, notamment de cellules sanguines, par exemple de cellules cancéreuses dans un échantillon de sang ou de globules, de bactéries, d'émulsions colloïdales ou non colloïdales, de protéines, de liposomes, méthodes de diagnostic ou d'analyse, méthodes de purification, d'enrichissement ou d'appauvrissement d'espèces, méthodes de synthèse d'espèces, méthodes de modification de caractéristiques physiques ou chimiques d'espèces, méthodes de recherche de médicaments, méthodes de mélange ou méthodes de mesure de coefficient de diffusion.

**Patentansprüche**

1. Vorrichtung (1) zur Handhabung von Objekten (O), die in einem Kanal (2) in einem Fluid (F), insbesondere einer Flüssigkeit, vorhanden sind, aufweisend:

- einen Kanal (2), der sich gemäß einer Längsachse (X) erstreckt, wobei der Kanal (2) einen Querschnitt mit einer gemäß einer ersten transversalen Achse (Y) gemessenen Breite (L) und einer gemäß einer zweiten, zur ersten senkrechten transversalen Achse (Z) gemessenen Dicke (e) hat, wobei die Breite (L) größer oder gleich der Dicke (e) ist, wobei der Kanal gemäß der zweiten transversalen Achse (Z) eine erste (3) und zweite (4) Wand aufweist,
- einen Schallwellenerzeuger (10), der in dem Kanal ab mindestens einer der Wände (3; 4) Schallwellen erzeugt,

**dadurch gekennzeichnet, dass**:

der Schallwellenerzeuger (10) ausgelegt ist, um eine Frequenz f, die sich von einer Resonanz-

frequenz $f_0$ des Kanals (2) unterscheidet, entlang der zweiten transversalen Achse (Z) bereitzustellen,

der Schallwellenerzeuger (10) ein Breitband-Schallwellenerzeuger ist, und

der Schallwellenerzeuger (10) die Resonanzfrequenz $f_0$ des Kanals (2) entlang der zweiten transversalen Achse (Z) nicht verwendet.

2. Vorrichtung (1) nach Anspruch **1,** wobei der Kanal (2) über mindestens einen Abschnitt seiner Länge, insbesondere über die Gesamtheit seiner Länge, eine Dicke (e) kleiner als 3 cm, insbesondere kleiner als 1 cm hat, wobei der Kanal (2) vorzugsweise ein Mikro-Kanal ist.

3. Vorrichtung (1) nach einem der Ansprüche **1** und **2,** wobei der Kanal (2) über mindestens einen Abschnitt seiner Länge, insbesondere über die Gesamtheit seiner Länge, einen rechteckigen Querschnitt aufweist.

4. Vorrichtung (1) nach einem der vorangehenden Ansprüche, die eine Vielzahl von Schallwellengeneratoren (10) aufweist, die entlang des Kanals angeordnet sind und in dem Kanal Schallwellen ab mindestens einer der Wände (3; 4) erzeugen, wobei die Schallwellengeneratoren (10) insbesondere auf derselben Seite des Kanals (2) angeordnet sind.

5. Vorrichtung (1) nach einem der vorangehenden Ansprüche, wobei mindestens eine der Wände (3; 4), vorzugsweise beide, aufweisen, insbesondere bestehen aus, einem Material, das ausgewählt ist aus: Mineralglas oder organischem Glas, thermoplastischen Materialien, insbesondere Polymethylmethacrylat oder Polycarbonat, Quarz, Metallen, dessen Produkt (Dichte des Metalls x Geschwindigkeit des Tons in diesem Metall) größer oder gleich $10^6$ Pa.s/m ist.

6. Vorrichtung (1) nach einem der vorangehenden Ansprüche, wobei mindestens eine Matte (N) aus Objekten (O) durch akustische Fokussierung gebildet wird, wobei insbesondere ein Schalldruckextremum ($E_p$) innerhalb des Fluids (F) von den erzeugten Schallwellen gebildet wird, und die Matte (N) aus Objekten (O) im Bereich des Schalldruckextremums ($E_p$) fokussiert wird.

7. Vorrichtung nach einem der vorangehenden Ansprüche, wobei der Schallwellenerzeuger (10) in einer Frequenz f arbeitet, die von $f_0$ unterschiedlich ist und zwischen 0,75 $f_0$ und 1,25 fo, insbesondere zwischen 0,75 $f_0$ und 0,95 $f_0$ oder zwischen 1,05 $f_0$ und 1,25 $f_0$ liegt.

8. Vorrichtung (1) nach Anspruch **7,** wobei die erste und zweite Wand ein Material mit einer akustischen Impedanz aufweisen, die mindestens zehn Mal höher als die des Fluids ist.

9. Vorrichtung (1) nach einem der vorangehenden Ansprüche, wobei der Kanal (2) eine Vielzahl von Ausgängen ($S_1$, ..., $S_n$) aufweist, zu denen die Objekte (O) gemäß der Frequenz f, in der der Schallwellenerzeuger (10) arbeitet, selektiv gelenkt werden.

10. Anordnung (200), aufweisend:

    - eine Vorrichtung (1) nach einem der Ansprüche **1** bis **9,**
    - einen Sensor (100), der erlaubt, mindestens ein Merkmal und/oder die Position der Objekte (O) im Kanal (2) zu messen, wobei der Sensor (100) in Abhängigkeit vom Ergebnis dieser Messung ein Signal erzeugt, und
    - ein Steuersystem (T), das das Signal empfängt und die Frequenz f, in der der Schallwellenerzeuger (10) arbeitet und/oder die Amplitude der in Abhängigkeit von dem Signal erzeugten Schallwellen steuert.

11. Anordnung (300), aufweisend:

    - eine Vorrichtung (1) nach einem der Ansprüche **1** bis **9,**
    - ein Beleuchtungssystem (110), das ausgelegt ist, um mindestens einen Teil der im Kanal (2) vorhandenen Objekte (O) zu beleuchten, und
    - ein Bilderfassungssystem (120), das ausgelegt ist, um mindestens ein Bild von mindestens einem Teil der im Kanal (2) vorhandenen und von dem Beleuchtungssystem (110) beleuchteten Objekte (O) aufzunehmen,
    wobei die Anordnung insbesondere eine Verarbeitungsvorrichtung (130) des mindestens einen vom Erfassungssystem (120) erfassten Bildes aufweist,
    wobei die Verarbeitungsvorrichtung (130) erlaubt, die Norm und/oder die Richtung und/oder den Sinn des Geschwindigkeitsvektors von mindestens einem Teil der im Kanal (2) vorhandenen und von dem Beleuchtungssystem (110) beleuchteten Objekte (O) zu messen.

12. Verfahren zur Handhabung von Objekten (O) in einem Kanal (2) mit Hilfe eines Schallwellenerzeugers (10), das eine Vorrichtung (1) nach einem der Ansprüche **1** bis **9** oder eine Anordnung nach einem der Ansprüche **10** bis **11** verwendet, wobei bei dem Verfahren:

    - sich der Kanal (2) gemäß einer Längsachse (X) erstreckt und einen Querschnitt mit einer gemäß einer ersten transversalen Achse (Y) gemessenen Breite (L) und einer gemäß einer zweiten, zur ersten senkrechten transversalen Achse (Z) gemessenen Dicke (e) hat, wobei die Breite (L) größer oder gleich der Dicke (e) ist, wobei der Kanal gemäß der zweiten transversalen Achse (Z) eine erste (3) und zweite (4) Wand aufweist, und

- der Schallwellenerzeuger (10) in dem Kanal ab mindestens einer der Wände (3; 4) Schallwellen erzeugt und in einer Frequenz f arbeitet, die sich von einer Resonanzfrequenz $f_0$ des Kanals (2) entlang der zweiten transversalen Achse (Z) unterscheidet,

- mindestens ein Merkmal und/oder die Position der im Kanal (2) vorhandenen Objekte (O) von einem Sensor (100) gemessen wird,

- ein Signal in Abhängigkeit vom Ergebnis der von dem Sensor (100) durchgeführten Messung erzeugt und zu einem Steuersystem (T) befördert wird, und

- die Frequenz f, in der der Schallwellenerzeuger (10) arbeitet und/oder die Amplitude der erzeugten Schallwellen in Abhängigkeit vom Signal durch Aktion des Steuersystems (T) verändert wird.

13. Verfahren zur Erfassung von mindestens einem Bild von in einem Kanal (2) vorhandenen Objekten (O), das die folgenden Schritte aufweist:

   a) Handhaben der Objekte (O) durch Durchführung eines Verfahrens nach Anspruch **12,** um eine akustische Fokussierung der Objekte (O) im Bereich einer bestimmten Zone des Kanals (2) zu erhalten,

   b) Beleuchten der Objekte (O) im Bereich der Zone der akustischen Fokussierung über ein Beleuchtungssystem (110), und

   c) Erfassen, über ein Erfassungssystem (120), mindestens eines Bildes der derart beleuchteten Objekte (O).

14. Verfahren zur Messung der Norm und/oder der Richtung und/oder des Sinns des Geschwindigkeitsvektors von Objekten (O) in einem Kanal (2), das die folgenden Schritte aufweist:

   - Erfassen eines ersten Bildes der Objekte (O) in einem ersten Moment durch Durchführung des Verfahrens nach vorangehendem Anspruch,

   - Erfassen eines zweiten Bildes der Objekte (O) in einem zweiten Moment durch Durchführung des Verfahrens nach vorangehendem Anspruch, und

   - Berechnen, ausgehend von dem ersten und zweiten Bild, eines Maßes der Norm und/oder der Richtung und/oder des Sinns des Geschwindigkeitsvektors der Objekte (O).

15. Verfahren nach einem der Ansprüche **12** bis **14, dadurch gekennzeichnet, dass** es in mindestens einer der folgenden Anwendungen verwendet wird: Particle Image Velocimetry ("PIV"), insbesondere Micro Particle Image Velocimetry ("micro-PIV"), Methoden zur Sortierung von Arten, beispielsweise von festen oder deformierbaren Partikeln, von polydispergierten Partikeln, von biologischen Zellen, insbesondere von Blutzellen, beispielsweise von Krebszellen in einer Blutprobe oder von Blutkörperchen, von Bakterien, von kolloidalen oder nicht kolloidalen Emulsionen, von Proteinen, von Liposomen, Diagnose- oder Analysemethoden, Reinigungs-, Anreicherungs- oder Abreichungsmethoden von Arten, Methoden zur Synthese von Arten, Methoden zur Veränderung physikalischer oder chemischer Merkmale von Arten, Methoden zur Erforschung von Arzneimitteln, Methoden zur Mischung oder Methoden zur Messung eines Diffusionskoeffizienten.

## Claims

1. Device (1) for handling objects (O) present in a channel (2) within a fluid (F), in particular a liquid, comprising:

   - a channel (2) extending along a longitudinal axis (X), the channel (2) having a cross-section with a width (L) measured along a first transverse axis (Y) and a thickness (e) measured along a second transverse axis (Z) perpendicular to the first, the width (L) being greater than or equal to the thickness (e), the channel comprising, along the second transverse axis (Z), first (3) and second (4) walls,
   - an acoustic waves generator (10) generating acoustic waves in the channel from at least one of said walls (3; 4),

   **characterized in that**:

   said acoustic waves generator (10) is configured to provide a frequency f different from a resonance frequency $f_0$ of the channel (2) along the second transverse axis (Z), said acoustic waves generator (10) is a broadband acoustic waves generator, and said acoustic waves generator (10) does not use the resonant frequency $f_0$ of the channel (2) along the second transverse axis (Z).

2. Device (1) according to claim 1, wherein the channel (2) has, over at least a portion of its length, in particular over its entire length, a thickness (e) of less than 3 cm, in particular less than 1 cm, the channel (2) preferably being a microchannel.

3. A device (1) according to any one of claims 1 and 2, wherein the channel (2) has, over at least a portion of its length, in particular over its entire length, a rectangular cross-section.

4. Device (1) according to any one of the preceding claims, comprising a plurality of acoustic waves generators (10) arranged along the channel and generating acoustic waves in the channel from at least one of the walls (3; 4), said acoustic waves generators (10) being in particular arranged on the same side of the channel (2).

5. Device (1) according to any one of the preceding claims, wherein at least one of the walls (3; 4), preferably both, comprises, in particular consists of, a material chosen among: mineral or organic glasses, thermoplastic materials, in particular polymethylmethacrylate or polycarbonate, quartz, metals whose product (density of the metal x speed of sound in this metal) is greater than or equal to $10^6$ Pa.s/m.

6. Device (1) according to any one of the preceding claims, wherein at least one web (N) of objects (O) is formed by acoustic focusing, in particular at least one acoustic pressure extremum (Ep) is formed within the fluid (F) by the generated acoustic waves, and the web (N) of objects (O) is focused at the acoustic pressure extremum (Ep).

7. Device according to any one of the preceding claims, wherein the acoustic waves generator (10) operates at a frequency f different from $f_0$ and ranging from $0.75f_0$ to $1.25f_0$, in particular from $0.75f_0$ to $0.95f_0$ or from $1.05f_0$ to $1.25f_0$.

8. The device (1) of claim 7, wherein the first and second walls comprise a material having an acoustic impedance at least ten times that of the fluid.

9. Device (1) according to any one of the preceding claims, wherein the channel (2) comprises a plurality of outlets (S1,..., Sn) towards which the objects (O) are selectively directed according to the frequency f at which the acoustic waves generator (10) operates.

10. An assembly (200) comprising:

   - a device (1) according to any one of claims 1 to 9,
   - a sensor (100) for measuring at least one characteristic and/or the position of the objects (O) present within the channel (2), said sensor (100) generating, as a function of the result of this measurement, a signal, and
   - a control system (T) receiving said signal and controlling the frequency f at which the acoustic waves generator (10) operates and/or the amplitude of the generated acoustic waves as a function of said signal.

11. An assembly (300) comprising:

   - a device (1) according to any one of claims 1 to 9,
   - an illumination system (110) configured to illuminate at least a portion of the objects (O) present within the channel (2), and
   - an image acquisition system (120) configured to acquire at least one image of at least part of the objects (O) present within the channel (2) and illuminated by the illumination system (110), said assembly comprising in particular a device (130) for processing the at least one image acquired by the acquisition system (120), the processing device (130) making it possible to measure the norm and/or the direction and/or the sense of the velocity vector of at least a part of the objects (O) present within the channel (2) and illuminated by the illumination system (110).

12. A method of manipulating objects (O) present in a channel (2) by means of an acoustic waves generator (10), using a device (1) according to any one of claims 1 to 9 or an assembly according to any one of claims 10 to 11, in which:

   - said channel (2) extends along a longitudinal axis (X) and has a cross-section with a width (L) measured along a first transverse axis (Y) and a thickness (e) measured along a second transverse axis (Z) perpendicular to the first, the width (L) being greater than or equal to the thickness (e), the channel comprising, along the second transverse axis (Z), first (3) and second (4) walls, and
   - said acoustic waves generator (10) generates acoustic waves in the channel from at least one of the walls (3; 4) and operates at a frequency f different from a resonance frequency $f_0$ of the channel (2) along the second transverse axis (Z),
   - at least one characteristic and/or the position of the objects (O) present within the channel (2) is measured by a sensor (100),
   - a signal is generated as a function of the result of the measurement performed by said sensor (100) and is sent to a control system (T), and
   - the frequency f at which the acoustic waves generator (10) operates and/or the amplitude of the generated acoustic waves is changed as a function of said signal by action of the control system (T).

**13.** A method of acquiring at least one image of objects (O) present within a channel (2) comprising the steps of:

a) manipulating the objects (O) by implementing a method according to claim 12 in order to obtain an acoustic focusing of said objects (O) at a determined zone of the channel (2),
b) illuminating the objects (O) at the level of the acoustic focusing zone by means of an illumination system (110), and
c) acquiring, by means of an acquisition system (120), at least one image of said objects (O) thus illuminated.

**14.** A method of measuring the norm and/or the direction and/or the sense of the velocity vector of objects (O) present within a channel (2) comprising the steps of:

- acquiring a first image of the objects (O), at a first instant, by implementing the method according to the preceding claim,
- acquiring a second image of the objects (O), at a second instant, by implementing the method according to the preceding claim, and
- calculating from the first and second images a measure of the norm and/or the direction and/or the sense of the velocity vector of the objects (O).

**15.** The method according to any one of claims 12 to 14, **characterized in that** it is used in at least one of the following applications: particle image velocimetry ("PIV"), in particular micrometric particle image velocimetry ("micro-PIV"), methods for sorting species, for example rigid or deformable particles, polydisperse particles, biological cells, in particular blood cells, for example cancer cells in a blood or blood cell sample, bacteria, colloidal or non-colloidal emulsions, proteins, liposomes, diagnostic or analytical methods, methods for purifying, enriching or depleting species, methods for synthesizing species, methods for modifying physical or chemical characteristics of species, methods for drug discovery, mixing methods or methods for measuring diffusion coefficients.

Fig. 1

Fig. 2

Fig. 3

Fig. 4

EP 2 750 778 B1

Fig. 5

Fig. 6

20

Fig. 7

Fig. 8

Fig. 9

Fig. 10

Fig. 11

Fig. 12

Fig. 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006095117 A
- WO 9817373 A
- WO 02072234 A
- WO 2009071733 A
- WO 2006032703 A
- WO 2004030800 A

**Littérature non-brevet citée dans la description**

- **DRON et al.** Parametric study of acoustic focusing of particles in a micro-channel in the perspective to improve micro-PIV measurements. *Microfluid Nano-fluid,* 2009, vol. 7, 857-867
- **GLYNNE-JONES et al.** Mode-switching: A new technique for electronically varying the agglomération position in an acoustic particle manipulator. *Ultrasonics,* 2010, vol. 50, 68-75
- **SVENNEBRING et al.** Selective Bioparticle Rétention and Characterization in a Chip-Integrated Confocal Ultrasonic Cavity. *J. Biotech. Bioeng,* 2009, vol. 103, 323-328
- **PETERSSON et al.** Séparation of lipids from blood utilizing ultrasonic standing waves in microfluidic channels. *Analyst,* 2004, vol. 129, 938-943
- **LIPKENS et al.** The Effect of Frequency Sweeping and Fluid Flow on Particle Trajectories in Ultrasonic Standing Waves. *IEEE Sensors Journal,* Juin 2008, vol. 8 (6), 667-677